# EUROPEAN PATENT APPLICATION

(11) **EP 1 964 836 A2**
(43) Date of publication of application: **03.09.2008**
(21) Application number: 07119678.6
(22) Date of filing: 30.10.2007
(51) Int. Cl.: C07D 209/42, C07K 5/06, A61K 31/404

(54) **A process for the preparation of perindopril erbumine in alpha crystalline form**

(30) Priority: 06.11.2006 IN MU18432006
(71) Applicant: IPCA Laboratories Limited, 400 067 Mumbai (IN)
(72) Inventor: Kumar, Ashok, Kandivli (West) 400067 Mumbai (IN); Soudagar, Satish Rajanikant, Kandivli (West) 400067 Mumbai (IN); Mathur, Arpana, Kandivli (West) 400067 Mumbai (IN); Panda, Nalinakshya Balaram, Kandivli (West) 400067 Mumbai (IN)
(74) Representative: Brand, Thomas Louis

(57) **Abstract**

The present invention concerns processes for the production of high purity perindopril erbumine in alpha crystalline form, perindoril erbumine being in a class of medications known as angiotensin-converting enzyme (ACE) inhibitors.

## Description

The present invention relates to processes for the production of high purity perindopril erbumine in alpha crystalline form, which is in a class of medications called angiotensin-converting enzyme (ACE) inhibitors

### Background Of The Invention

Perindopril (Formula IA) and its pharmaceutically acceptable salts, especially the *tert.* butylamine salt (Formula IB), is an inhibitor of the enzyme that converts angiotensin I (or kininase II), a precursor for formation of angiotensin II enzyme, thereby enables on the one hand prevention of the conversion of the decapeptide angiotensin I to the octapeptide angiotensin II (vasoconstrictor) and on the other hand prevention of the degradation of bradykinin (vasodilator) to inactive peptide. These two actions contribute to the beneficial effects of perindopril or its salts in cardiovascular disorders, especially arterial hypertension and cardiac insufficiency. The use of perindopril in these therapies demands high purity of the final compound in a manufacturing operation.

Perindopril, its preparation and its therapeutic use were first described in European Patent Specification No. 0049658. There are ample literatures available for the preparation of perindopril and its erbumine salt exploring various synthetic alternatives. However there are only a few reports on the production of a stable crystalline form of perindopril erbumine. Among them WO0187835, WO0187836 & WO0183439 patents disclose that perindopril erbumine can exist in three different polymorphic forms (designated as Form alpha and Form beta and Form gamma) and provides analytical characterization for those polymorphs. Here it is worth mentioning that at least two of these polymorphs (alpha & beta) were isolated from the same solvent - ethyl acetate, however, by applying different processing conditions. However it is also reported that preparation of specific alpha form of perindopril erbumine from ethyl acetate is not consistently reproducible. Patent publication No.WO/2005/037788 discloses a polymorph referred to as not falling in alpha, beta or gamma form and process for obtaining such a form from different solvents. Patent application No. US20050250706 (Glenmark pharmaceuticals) discusses various alternative solvents for preparation of perindopril erbumine in alpha form. This patent makes use of mainly ketonic solvents or its mixture with nitriles for the preparation of alpha form.

### Summary of the Invention

The present inventors had discovered that the prior art processes present substantial difficulties in producing alpha crystal form of perindopril erbumine in a consistent manner. The invention, therefore, aims to provide an improved process for making Form alpha of perindopril erbumine. In accordance with one aspect, the invention provides a process for the preparation of crystalline Form alpha of perindopril erbumine, which process includes isolating perindopril erbumine in crystalline "Form alpha" from a solvent selected from C5-C10 hydrocarbon solvents (aromatic or aliphatic) or its mixtures with esters like ethyl acetate; primary or secondary or tertiary alcohols; mixture of esters with alcohols like 1-butanol; nitriles such as acetonitrile; ethers such as methylcellosolve, dioxane; ketoesters such as ethyl acetoacetate; dipolar solvents like Dimethylformamide, dimethylsulphoxide and the like. Preferably the solvent for isolation is benzene, toluene, xylene or mixture of toluene & ethyl acetate or mixture of ethyl acetate & benzene under suitable conditions.
The details of one or more embodiments of the inventions are set forth in the description below. Other features, objects and advantages of the inventions will be apparent from the appended examples and claims.

### Detailed Description Of The Invention

Unless specified otherwise, all technical and scientific terms used herein have the same meaning as commonly understood by one of ordinary skill in the art, to which this invention belongs. Although any methods and materials similar or equivalent to those described herein can be used in the practice or testing of the present invention, the preferred methods and materials are described. To describe the invention, certain terms are defined herein specifically as follows. Unless stated to the contrary, any of the words "including," "includes," "comprising," and "comprises" mean "including without limitation" and shall not be construed to limit any general statement that it follows to the specific or similar items or matters immediately following it. Embodiments of the invention are not mutually exclusive, but may be implemented in various combinations. The described embodiments of the invention and the disclosed examples are given for the purpose of illustration rather than limitation of the invention as set forth the appended claims.

The term "isolating" is used to indicate separation or collection or recovery of the compound being isolated in the specified crystalline form.

The term "separating from a solvent" with respect to the crystalline solids described herein means obtaining a solid of specified characteristics from a solution or a partial solution.

The term "treating" means adding or combining or mixing the stated reagent or materials to the thing being treated.

The term "forming a solution" means obtaining a solution of a substance in a solvent in any manner.

The term "inoculating" has the same meaning as the term "seeding," and means adding previously obtained solid to facilitate crystallization. Thus, the term "seeding crystals" with respect to claimed process means crystals/powder of previously obtained crystalline Form alpha of perindopril erbumine.

The term "hydrocarbon" means solvent containing hydrocarbons. The term does not exclude solvents containing insignificant amounts of other solvents. "Perindopril erbumine" is a tertiary butyl amine salt of (2S, 3aS, 7aS)-1-[(2S)-2-[[(1S)-1-(ethoxycarbonyl) butyl] amino]-1-oxopropyl] octahydro-1*H*-indole-2-carboxylic acid. It has the structural formula: 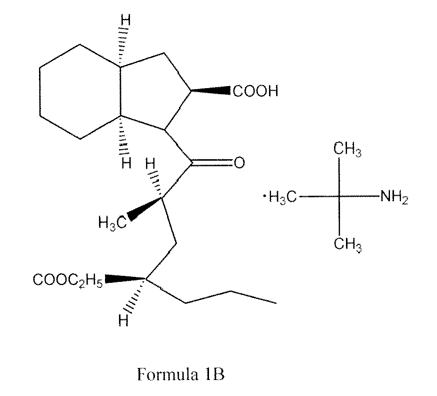 "Perindopril or perindopril free acid" is a free species of (2S, 3aS, 7aS)-1-[(2S)-2-[[(1S)-1-(ethoxycarbonyl) butyl] amino]-1-oxopropyl] octahydro-1 H-indole-2-carboxylic acid. It has the formula:

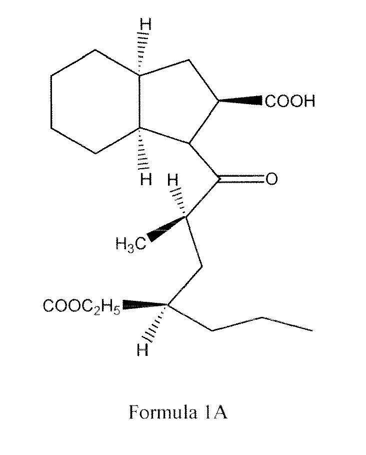

Polymorphic forms of perindopril erbumine are known. See, e.g., PCT applications WO0187835, WO0187836 & WO01834395. These publications disclose and characterize three polymorphic forms of the drug: Form alpha, Form beta and Form gamma. The WO0187835 publication describes that the perindopril was not obtained in a stable crystalline form consistently for pharmaceutical applications before.

For the purposes of this description and claims of the present invention, the term "crystalline Form alpha of perindopril erbumine" is the polymorphic form denoted as Form alpha in the WO0187835 patent. The WO0187835 patent publication herein also incorporated by reference, is specifically for the purposes of providing the reference analytical information (XRD) for Forms alpha, beta and gamma. Identification of solids obtained by the process of the invention can be made by comparing with the reference analytical information provided in the WO0187835 patent. Of course, it should be understood that operator, instrument and other similar issues may result in some margin of error with respect to analytical characterization of the solid.

The WO0187835 describes isolation of crystalline Form alpha of perindopril by dissolution of perindopril erbumine in ethyl acetate by heating until complete dissolution, filter the solution followed by gradual cooling of the ethyl acetate solution to about 60°C with a cooling rate of about 5-10°C per hour and thereafter cooled to room temperature that leads to precipitation of the perindopril erbumine salt in alpha crystal form. To obtain Form Beta from the same solvent, a rapid cooling was applied. The inventors of the present invention had found that the use of ethyl acetate does not provide a reliable, consistent methodology to prepare Form alpha. The inventors had recognized that in using ethyl acetate small changes in manufacturing parameters might lead to contamination of the desired solid Form alpha with Form beta impurities. The inventors, on exploring various process alternatives, for a reliable process solution have found that the use of hydrocarbons or its mixture with ethyl acetate and alcohols or mixtures of ethyl acetate and dioxane as a solvent for isolation permits reliable preparation of Form alpha of perindopril erbumine. The especially important solvent or solvent combinations include C5-C10 hydrocarbon solvents (aromatic or aliphatic) or its mixtures with esters like ethyl acetate; primary or secondary or tertiary alcohols; mixture of esters with alcohols like 1-butanol; nitriles such as acetonitrile; ethers such as methylcellosolve, dioxane; ketoesters such as ethyl acetoacetate; dipolar solvents like Dimethylformamide, dimethylsulphoxide; isopropyl acetate or the like. Preferably the solvent for isolation is benzene, toluene, xylene or mixture of toluene & ethyl acetate or mixture of ethyl acetate & benzene under suitable conditions. Especially preferred hydrocarbons are toluene, benzene and xylene and alcohol is n-butanol. Among ester, isopropyl acetate is preferred or its combinations with alcohols or hydrocarbons; mixture of toluene & ethyl acetate; or mixture of ethyl acetate & benzene are preferred.

Thus, Form alpha of perindopril erbumine may be obtained from a solution of perindopril erbumine in benzene, toluene, xylene, cyclohexane, isopropyl acetate, acetonitrile, 1,4-dioxane, methylcellosolve, ethylacetoacetate, isobutyl alcohol, dimethyl formamide, dimethylsulphoxide, ethylacetate-toluene, ethylacetate-benzene, ethylacetate - n-butanol, benzene - n-butanol, toluene-n-butanol or any similar cross combinations.

According to a preferred embodiment, the preparation of perindopril erbumine in form alpha comprises the steps of:
a. forming a solution of perindopril or its salt in the solvents as mentioned above;
b. optionally adding tertiary butylamine to said solution;
c. crystallizing perindopril erbumine in alpha form; and
d. separating alpha crystals of perindopril erbumine from the solution.

The solution of perindopril erbumine may be prepared by mixing perindopril erbumine in the solvent of choice and stirring for dissolution. Preferably a solution is formed under heating. The solution is then allowed to cool to precipitate the perindopril erbumine in alpha crystalline form which can be separated from the solvent by filtration.

Alternately, the perindopril erbumine solution may be formed by dissolving perindopril free acid in the solvent and the solution may then be filtered to remove any particulate matter. Once the solution of the perindopril acid is obtained, tertiary butyl amine is added to form the perindopril erbumine salt. The mass may be heated till complete dissolution. The mass is then cooled until crystallization of the solid is complete. The solid is filtered, washed, and dried. In the process, optionally, either before or after tertiary butyl amine addition, the solution may be seeded with previously obtained crystals of the Form alpha.
Alternatively, the process for alpha form can also include equilibrating or slurrying perindopril erbumine in any solid form in one or more of the solvents defined above for a period sufficient to obtain alpha form.

The starting perindopril free acid or perindopril erbumine may be obtained by following any known process disclosed in the literature. The present inventors used samples obtained as per the process disclosed in EP1670072.

Analytical characterization of the solid(s) obtained in accordance with the process of the invention was carried out by using X-ray powder diffraction using a PANALYTICAL XpertPRO X-Ray machine of Philips make. The X-ray powder diffraction patterns were recorded with Cu K alpha-1 radiation source (voltage of 50 kV; current: 25 mA). The analytical data obtained for the solids were compared with data provided in the WO0187835 patent.

The stable Alpha form of perindopril erbumine obtained by the process of the present invention may be formulated into a dosage form, e.g., tablet, capsule, etc., by combining with one or more pharmaceutically acceptable excipients using known techniques. The resulting dosage form may include a suitable amount of the active ingredient and a diuretic such as indapamide. For example, the resulting dosage form may contain between 3 and 50 mg of perindopril erbumine alpha form. Further, the dosage form may be immediate release or extended release. The dosage forms may be administered to a mammal in need, for ameliorating antihypertesion or cardiovascular diseases.

The examples provided below are illustrative and are not intended to limit the scope of the claimed invention.

### Examples

### Example 1.

2.0 grams of perindopril erbumine was taken in 18 ml benzene at room temperature. It was then heated until complete dissolution at 65°C. The solution was then cooled to about 30°C and the precipitated crystals were filtered, washed with fresh benzene and dried under vacuum at 30°C. to constant weight. Yield 1.9 gm. The XRPD of the sample was recorded and found matching with Form alpha.

### Example 2.

2.0 grams of perindopril erbumine was taken in a mixture of 30 ml ethyl acetate and 8 ml 1-butanol at room temperature. It was then heated until complete dissolution at 75°C. The solution was then cooled to about 10°C and the precipitated crystals were filtered and washed with fresh ethyl acetate and dried under vacuum at 25-30°C. to constant weight. Yield 1.2 gm. The XRPD of the sample was recorded and found matching with Form alpha.

### Example 3.

2.0 grams of perindopril erbumine was taken in 14 ml toluene at room temperature. It was then heated until complete dissolution at 75°C. The solution was then filtered, and the filtrate cooled to about 30°C and the precipitated crystals were filtered, washed with fresh toluene and dried under vacuum at 30°C. to constant weight. Yield 1.9 gm. The XRPD of the sample was recorded and found matching with Form alpha.

### Example 4

2.0 grams of perindopril erbumine was taken in 14 ml ethylacetoacetate at room temperature. It was then heated until complete dissolution. The solution was then filtered, and the filtrate cooled to about 10°C. The precipitated crystals were filtered, and dried under vacuum at 40°C. to constant weight. Yield 0.6 gm. The XRPD of the sample was recorded and found matching with Form alpha.

### Example 5.

2.0 grams of perindopril erbumine was taken in 20 ml dimethylformamide (DMF) at room temperature. It was then heated until complete dissolution. The solution was then filtered, and the filtrate cooled to about 30°C. The precipitated crystals were filtered and dried under vacuum at 40°C to constant weight. Yield 1.7 gm. The XRPD of the sample was recorded and found matching with Form alpha.

### Example 6.

2.0 grams of perindopril erbumine was taken in 20 ml dimethyl sulphoxide (DMSO) at room temperature. It was heated to about 65°C to dissolve. The solution was then filtered, and the filtrate cooled to about 30°C. The precipitated crystals were filtered and dried. Yield 1.7 gm. The XRPD of the sample was recorded and found matching with Form alpha.

### Example 6.

1.0 grams of perindopril erbumine (a mixture of different polymorphs) was taken in 10 ml heptane and agitated at 30°C for about 48 hours. The suspension was then filtered, and the obtained crystals were dried under vacuum. Yield 0.9 gm. The XRPD of the sample was recorded and found matching with Form alpha.

## Claims

1. A process for the preparation of perindopril erbumine of Formula IB in alpha crystalline form comprising isolating the alpha crystalline form in a solvent or solvent mixture selected from one or more of: aliphatic and/or aromatic C₅-C₁₀ hydrocarbon solvents or mixtures of two or more thereof; mixtures of one or more hydrocarbons with one or more esters; primary, secondary or tertiary alcohols and mixtures of two or more thereof; mixtures of one or more esters with one or more alcohols; nitriles; ethers; ketoesters; dipolar solvents; and isopropyl acetate.

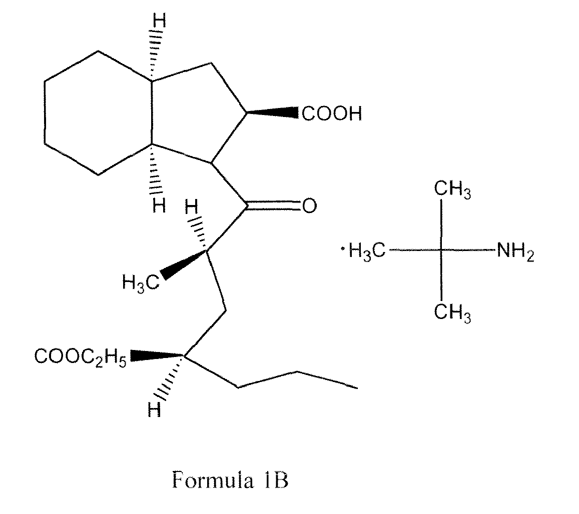

2. A process according to claim 1 wherein the solvent or solvent mixture is selected from benzene, toluene, xylene, cyclohexane, isopropyl acetate, acetonitrile, 1,4-dioxane, methylcellosolve, ethylacetoacetate, isobutyl alcohol, dimethyl formamide, dimethylsulphoxide, ethylacetate-toluene, ethylacetate-benzene, ethylacetate - n-butanol, benzene - n-butanol, toluene-n-butanol and combinations of two or more thereof.

3. A process according to claim 2 wherein the solvent is toluene and/or xylene.

4. A process according to any one of claims 1 to 3 comprising slurrying or equilibrating perindopril erbumine of any solid form or mixture of solid forms in the solvent or solvent mixture.

5. A process according to any one of claims 1 to 4 wherein the perindopril salt is tertiary butyl amine salt.

6. A process according to any one of claims 1 to 5 comprising;
a. forming a solution of perindopril or its salt in the solvent or solvent mixture;
b. optionally adding tertiary butylamine to said solution;
c. crystallizing perindopril erbumine in alpha form; and
d. separating alpha crystals of perindopril erbumine from the solution.

7. A process according to claim 6 wherein step(a) is conducted under heating to effect complete dissolution of perindopril or its salt.

8. A process according to claim 6 or claim 7 wherein tertiary butyl amine is added in molar equivalent amounts with respect to perindopril when step (a) uses perindopril free acid.

9. A process according to claim 6 or claim 7 wherein tertiary butyl amine is added in excess, when perindopril salt is not an erbumine salt.

10. A according to any one of claims 6 to 9, wherein the crystallization is conducted by cooling to ambient temperature or below.

11. A process according to any one of claims 6 to 10 wherein a seed crystal of alpha perindopril erbumine is used during crystallization.

12. Perindopril erbumine in pure alpha form free from any contaminating polymorphs.

13. Perindopril erbumine alpha form substantially free of form 'beta' and form 'hydrated beta'.

14. Use of perindopril erbumine alpha form obtained according to any one of the preceding claims in a pharmaceutical composition.
